(19) Europäisches Patentamt
European Patent Office
Office européen des brevets

(11) EP 2 984 140 B1

(12) # EUROPEAN PATENT SPECIFICATION

(45) Date of publication and mention of the grant of the patent:
**13.07.2016 Bulletin 2016/28**

(21) Application number: **14726236.4**

(22) Date of filing: **14.03.2014**

(51) Int Cl.:
***C09B 61/00*** (2006.01) ***C07H 17/065*** (2006.01)
***A23L 5/42*** (2016.01)

(86) International application number:
**PCT/US2014/027385**

(87) International publication number:
**WO 2014/152478 (25.09.2014 Gazette 2014/39)**

(54) **NATURAL BLUE ANTHOCYANIN-CONTAINING COLORANTS**

FÄRBEMITTEL MIT NATÜRLICHEM BLAUEM ANTHOCYANIN

COLORANTS CONTENANT DE L'ANTHOCYANINE BLEUE NATURELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361790746 P**

(43) Date of publication of application:
**17.02.2016 Bulletin 2016/07**

(73) Proprietors:
- **Mars, Incorporated**
  **McLean, VA 22101 (US)**
- **The Ohio State University**
  **Columbus, OH 43201 (US)**

(72) Inventors:
- **ROBBINS, Rebecca J.**
  **Hackettstown, New Jersey 07840 (US)**
- **JOHNSON, J. Christopher**
  **Turlock, California 95382 (US)**
- **COLLINS, Thomas M.**
  **Hackettstown, New Jersey 07840 (US)**
- **AHMADIANI, Neda**
  **Columbus, Ohio 43201 (US)**
- **GIUSTI, M. Monica**
  **Columbus, Ohio 43201 (US)**

(74) Representative: **Greaves, Carol Pauline**
**C P Greaves & Co**
**80 Church Road**
**Winscombe**
**North Somerset BS25 1BP (GB)**

(56) References cited:
**WO-A1-2010/114568**
**WO-A2-2005/007088**
**WO-A2-2009/100165**
**WO-A2-2010/131049**
**WO-A2-2012/172429**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 984 140 B1

## Description

BACKGROUND OF THE INVENTION

Field of the Invention

The present invention relates to natural blue anthocyanin-containing colorants comprising fractions of anthocyanin molecules separated from anthocyanin-containing vegetable and fruit juices and extracts at a select pH based on differences in molecule charge and polarity.

DESCRIPTION OF THE RELATED ART

There is increasing interest in the food industry to replace synthetic materials for coloring foods with natural colorants.

One challenge in replacing synthetic colorants with natural colorants has been identifying natural colorants that provide color characteristics similar to those provided by synthetic colorants. Natural colorants that provide the same color characteristics as the synthetic blue colorant, FD&C Blue No. 1, have not been found, to this time. The lack of appropriate natural cyan blue hue colorants has also made it challenging to obtain desired natural green hue colorants from the blending of natural blue and yellow colorants.

Spirulina Blue, a blue-green algae-derived material, is used as a natural blue colorant, but does not provide the same color characteristics as FD&C Blue No. 1.

Anthocyanins are water-soluble compounds found in the cell vacuoles of fruits, vegetables, and flower petals, and sometimes, roots, leaves, stems, and bracts of plants. At least in part due to their wide availability, anthocyanin-containing vegetable and fruit juices and extracts have been used as natural, edible colorants and to produce colorants, in particular, natural red, purple, and blue hue colorants.

An anthocyanin comprises an anthocyanidin (the aglycone) esterified to one or more sugar molecules (the glycone(s)) to form a glycoside. Sugar molecules may be attached at the C-3, C-5, C-7, C-3', C-4', and/or C-5' positions. Examples of sugar molecules found in anthocyanin structures are arabinose, galactose, glucose, rhamnose, rutinose, sambubiose, sophorose, and xylose.

Anthocyanins may also be acylated, i.e., they may have one or more molecules esterified to the sugar molecules, typically at the 6-position of a monosaccharide, but also potentially at the 2-, 3-, or 4-positions. The most common acyl units include those derived from coumaric, ferulic, caffeic, sinapic, gallic, malonic, acetic, malic, succinic, vanillic, and oxalic acids.

The structure of an anthocyanidin is shown below in the flavylium cation form, which is the primary form under acidic conditions. The anthocyanidin may be substituted with hydrogen, hydroxyl, and/or methoxyl groups at various positions:

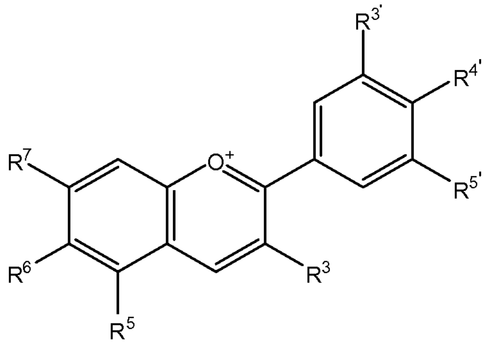

wherein

$R^3$ is H or OH,
$R^5$ is H, OH, or $OCH_3$,
$R^6$ is H or OH,
$R^7$ is OH or $OCH_3$,
$R^{3'}$ is H, OH, or $OCH_3$,
$R^4$ is OH or $OCH_3$, and
$R^{5'}$ is H, OH, or $OCH_3$.

The most common anthocyanidins in nature are shown by the following structures:

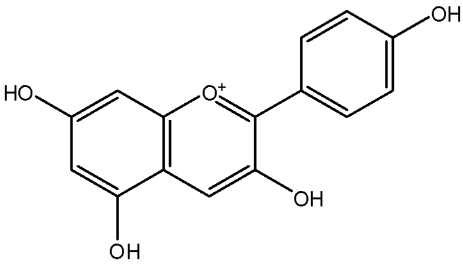

Pelargonidin

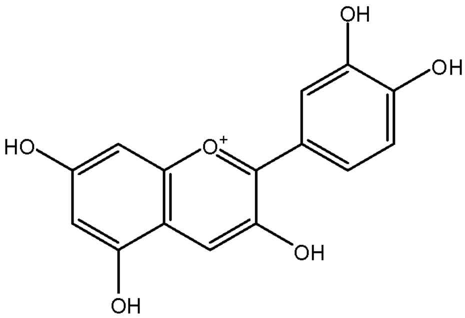

Cyanidin

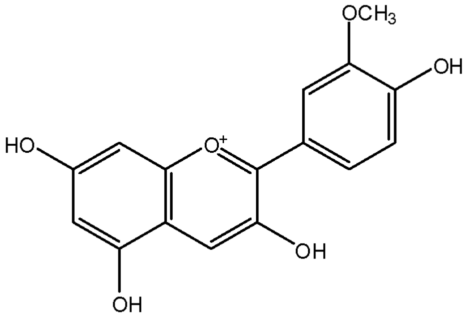

Peonidin

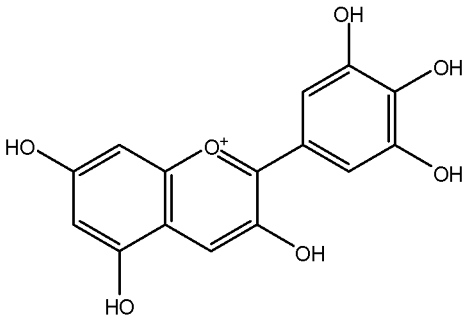

Delphinidin

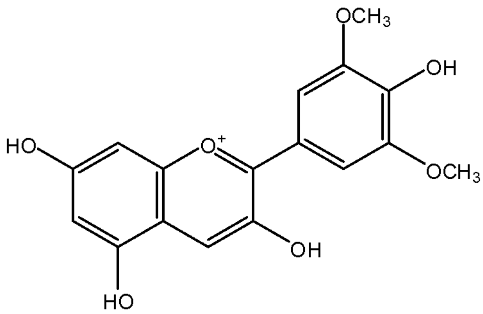

Malvidin

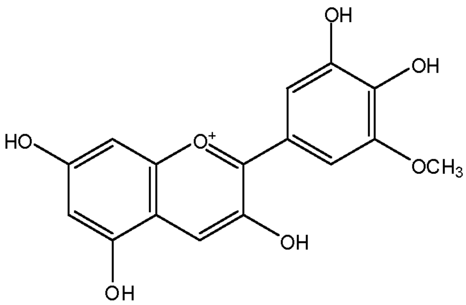

Petunidin

Therefore, the class of compounds known as anthocyanins encompasses an enormous number of structurally diverse compounds based on differences in primary structure, glycosylation and acylation patterns.

Known plant sources of anthocyanins include: (1) vegetables such as red cabbage, purple sweet potato, blue potato, red potato, red radish, black carrot, purple carrot, purple corn, red corn, red onion, purple broccoli, red broccoli, purple cauliflower, rhubarb, black bean, red leaf lettuce, black rice and eggplant; and (2) fruits such as strawberry, raspberry, cranberry, lingonberry, red grape, apple, black currant, red currant, cherry, blueberry, elderberry, bilberry, crowberry, blackberry, chokeberry, gooseberry, açaí, nectarine, peach, plum, blood orange and blue tomato. Each anthocyanin source contains different amounts of multiple, distinct anthocyanin species, with 15 to 30 structurally distinct anthocyanin molecules being common for a given plant source.

The color characteristics of anthocyanin-containing vegetable and fruit juices and extracts change as a result of changing pH. Anthocyanin-containing juices and extracts generally exhibit red hues at low pH, and the hue shifts to purple as the pH is increased. Only a few juices and extracts exhibit a blue hue as pH is increased further.

The change in color of anthocyanin-containing juices and extracts resulting from changes in pH is related to the numerous secondary structures of anthocyanins that may exist in equilibrium with the primary flavylium cation structure in aqueous solution. When pH is changed, the relative quantities of the different equilibrium structures will change. At a given pH, one or more structural forms may predominate, while others are present in low quantities or not present. For example, at very low pH, the flavylium cation form predominates. As pH is increased, molecules in the

flavylium cation form may be deprotonated and converted to the carbinol pseudobase form, which may be further converted through loss of a water molecule and a proton to the neutral and ionized quinonoidal base forms, respectively, and further, to the chalcone form. These transformations reduce the quantity of molecules in the flavylium cation form and increase the quantities in the other equilibrium forms to different extents. Therefore, the different equilibrium structures exist in different relative quantities at higher pH compared to low pH. Each structural form of anthocyanin may absorb light differently, resulting in a different perceived color, including no color. Therefore, as the pH of the solution is changed, changes in the relative quantities of the different structural forms may result in changes in the color of the solution.

Each distinct anthocyanin molecule is characterized by its own set of equilibrium molecular structures and equilibrium constants for the reactions that transform one structure into another. For example, the reaction transforming one anthocyanin equilibrium structure into another may have a particular acid dissociation constant, $K_a$, associated with it. The reaction may also be discussed in terms of the logarithmic constant, $pK_a$, which is defined as $-\log_{10} K_a$.

The flavylium cation and quinonoidal base structures have conjugated bonds connecting all three rings of the anthocyanin molecules. The extensive delocalized pi bonds allow the flavylium cation and quinonoidal base to absorb visible light, resulting in the perceived red hue of the flavylium cation at low pH and the purple or blue hue of the ionized quinonoidal base at a higher pH. In contrast, the carbinol pseudobase and chalcone structures do not have delocalized pi bonds connecting all three rings and are colorless or slightly yellow.

The substitution pattern of anthocyanins also affects color. For example, it is generally observed that the hue shifts from pink to purple when hydrogen atoms are replaced with hydroxyl groups. Similarly, the number of glycosyl (sugar) units and the number and type of acyl units are observed to affect color. However, these phenomena are not well understood or predictable.

Additionally, intermolecular and intramolecular interactions also affect anthocyanin color. The same anthocyanin may produce different hues depending on the other molecules present. For example, it is believed that acyl groups on the anthocyanin sugars can fold in and protect the flavylium cation C-2 position from nucleophilic attack. Therefore, this intramolecular interaction prevents formation of the colorless carbinol pseudo-base structure. Similarly, it is believed that anthocyanin molecules self-associate, which is evidenced by the fact that a two-fold increase in anthocyanin concentration can cause a 300-fold increase in chroma, and can change the hue and value as well. It is hypothesized that this self-association is similar to intramolecular stacking, and prevents nucleophilic attack and formation of the carbinol pseudo-base structure.

Although it is known that factors such as pH, anthocyanin chemical structure, substituent patterns, inter- and intra-molecular interactions all impact the color observed in anthocyanin-containing vegetable and fruit juices and extracts, it is not well understood how these factors interact to alter color, i.e., the specific cause and effect are not predictable.

For example, individual anthocyanin molecules have been separated by HPLC, but the separation has always occurred at low pH, and the color characteristics of individual anthocyanins were analyzed at low pH. Similarly, the effect of pH on the color characteristics of anthocyanin-containing vegetable and fruit juices and extracts has been studied, but these studies have analyzed the complex mixtures of anthocyanins naturally occurring in the juices and extracts. How changing pH affects the color characteristics of individual anthocyanin molecules or fractions of anthocyanins separated from natural sources, however, is not well understood or predictable. The prior art discloses that the number and types of substituents, e.g., the sugar and acyl groups, impact color; however, it does not disclose and it is not known how these substituents affect color as pH changes. Finally, although the prior art hypothesizes that various inter- and intra-molecular interactions affect color, it does not disclose how changing pH affects these inter- and intra-molecular interactions and, ultimately, the observed color of the anthocyanins.

WO 2009/100165 A2 discloses a method of separating anthocyanins from other phenolic molecules in the juice of anthocyanin-containing fruits and vegetables. WO 2009/100165 A2 does not disclose selectively separating fractions of anthocyanin molecules based on differences in charge and polarity of the molecules to produce fractions with a desired color that is different than the anthocyanin-containing juice.

The separation of individual anthocyanins at analytical scale is described in J. Chromatography A., 1148 (2007), 38-45. The separation is conducted at low pH, i.e., pH of less than 2, using HPLC in order to assist in identifying individual anthocyanins. This method separates anthocyanin molecules for detection rather than producing fractions with mixtures of anthocyanins.

WO 2004/012526 discloses a blue colorant solution of red cabbage anthocyanins at a pH of 7.9 that is used in a sugar-syrup for coating confectionery cores. The red cabbage anthocyanins were not separated into fractions. WO 2010/114568 A1 provides a stable blue food colorant on natural basis; a plant callus is cultivated, thereby a suspension cell culture is capable of producing blue anthocyanin with the help of a blue anthocyanin-generating agent, which comprises ammonium molybdate.

There is no example in the prior art of anthocyanin-containing colorants comprising fractions of anthocyanin molecules separated from anthocyanin-containing vegetable and fruit juices and extracts at a select pH based on differences in charge and polarity of the molecules. In addition, anthocyanin fractions that provide different color characteristics than those provided by the source juices and extracts have not been disclosed. In particular, the prior art has

not described natural blue anthocyanin-containing colorants providing color characteristics similar to those provided by the synthetic blue colorant, FD&C Blue No. 1.

It is desirable to have a broad palette of colorants available for coloring foods. In particular, there is a need for natural colorants that provide color characteristics similar to those provided by synthetic colorants, especially FD&C Blue No. 1. Anthocyanin fractions obtained from vegetable and fruit juices and extracts constitute a potential source of these natural colorants.

SUMMARY OF THE INVENTION

The present invention is directed to natural blue anthocyanin-containing colorants providing color characteristics similar to the synthetic blue colorant, FD&C Blue No. 1. The natural blue anthocyanin-containing colorant is obtained by selective separation of anthocyanins from anthocyanin-containing vegetable and fruit juices and extracts at a select pH based on differences in charge and polarity of the anthocyanin molecules.

In an embodiment, a natural blue anthocyanin-containing colorant is sourced from vegetable, fruit or combinations thereof, and comprises a selectively separated mixture of anthocyanins, wherein the colorant when in an aqueous solution at pH 8.0 has a maximum absorbance of 615 nm to 635 nm. The selectively separated mixture of anthocyanins is a subset of, i.e., a fraction or combination of fractions of the mixture of anthocyanins that exist in the vegetable or fruit in nature, and thus is different than mixtures of anthocyanin that exist in nature.

The present invention is also directed to natural green colorants produced by mixing the natural blue anthocyanin-containing colorant with a natural yellow colorant. A preferred natural yellow colorant is turmeric or curcumin.

The present invention is also directed to an edible product colored with the natural blue anthocyanin-containing colorant. The edible product may also include a natural yellow colorant to produce a green colored edible product.

BRIEF DESCRIPTION OF THE DRAWINGS

Figure 1 shows two perspectives of a three dimensional representation of the color characteristics provided by FD&C Blue No. 1 in CIE 1976 CIELAB L*a*b* color space as a function of concentration in aqueous solution.

Figure 2 shows two perspectives of a three dimensional representation of the color characteristics provided by FD&C Blue No. 1 in CIE 1976 CIELCH L*C*h° color space as a function of concentration in aqueous solution.

Figure 3 represents two perspectives of the area in CIE 1976 CIELAB L*a*b* color space of colors that differ from the colors provided by FD&C Blue No. 1 by a ΔE of 3 or less and an illustration of a segmented tube defined by the color space data.

Figure 4 shows a comparison of the colors provided by different fruit and vegetable extracts in aqueous solution at different pH values.

Figure 5 shows two perspectives of a three dimensional representation of the color characteristics provided by FD&C Blue No. 1 in CIE 1976 CIELAB L*a*b* color space as a function of concentration in aqueous solution as well as the area of colors that differ from the colors provided by Blue No. 1 by a ΔE of 3 or less and also shows two perspectives of a three dimensional representation of the color characteristics provided by Spirulina Blue as a function of concentration in aqueous solution (white line closer to the x-axis).

Figure 6 shows HPLC chromatograms at 520 nm detection of red cabbage extract solution and two fractions isolated from red cabbage extract solution using a strong cation exchange column.

Figure 7 shows HPLC chromatograms at 520 nm detection of red cabbage extract solution and four fractions isolated from red cabbage extract solution using a strong cation exchange column.

Figure 8 shows HPLC chromatograms at 520 nm detection of red cabbage extract solution identifying two groups of peaks that were targeted for isolating. These two groups of peaks were isolated as the "520-nm Fraction" and the "530-nm Fraction."

Figure 9 provides a visual comparison of the colors provided by the 520-nm and 530-nm Fractions at different pH values. Figure 9 also allows for a visual comparison of the colors provided by the 520-nm and 530-nm Fractions with the color of a confectionery product panned with a sugar-syrup colored with FD&C Blue No. 1.

Figure 10 shows HPLC chromatograms at 520 nm of red cabbage extract solution and two fractions isolated from red cabbage extract solution using semi-preparative HPLC. Figure 10 shows that the 520-nm and 530-nm Fractions each contain three distinct anthocyanin compounds and identifies the functional groups and sugars on the anthocyanin compounds.

DETAILED DESCRIPTION OF THE INVENTION

Anthocyanin-containing vegetable and fruit juices and extracts are presently used as natural, edible colorants and to produce colorants, in particular, natural red, purple, and blue hue colorants. The juices and extracts contain a mixture of all the anthocyanin molecules naturally present in the vegetable and fruit sources, along with numerous other classes of compounds. Therefore, the presently available anthocyanin-containing colorants are limited to those colors associated with the mixtures of anthocyanins that naturally exist in the vegetable and fruit sources. The invention involves natural blue anthocyanin-containing colorants that contain anthocyanins that are selectively separated from the mixture as it exists in nature so as to provide color characteristics similar to those provided by the synthetic blue colorant, FD&C Blue No. 1.

One aspect of the invention involves isolating fractions of anthocyanin molecules from anthocyanin-containing vegetable and fruit juices and extracts to obtain colorants providing specific, targeted color characteristics similar to those provided by the synthetic blue colorant, FD&C Blue No. 1. As used herein, providing color characteristics "similar" to FD&C Blue No. 1 means the color is closer in color characteristics than any other natural colorant, such as for example, Spirulina Blue.

The applicants discovered that separating anthocyanins using solvent at a select pH and differences in polarity of the anthocyanin molecules would yield fractions containing mixtures of anthocyanins providing color characteristics similar to those provided by the synthetic blue colorant, FD&C Blue No. 1. Each anthocyanin source contains different amounts of multiple, distinct anthocyanin molecules, and each molecule may exist in equilibrium with one or more secondary structures. There may be differences in charge and/or polarity among the different anthocyanin molecules and their equilibrium molecular structures. Through separation based on differences in charge and polarity of the anthocyanin molecules at a select pH, the applicants were able to isolate fractions of anthocyanins with distinct spectral characteristics from a complex mixture of anthocyanins. The spectral characteristics of the fractions were different and not evident from the spectral characteristics of the complex mixture of anthocyanins found in the juice or extract. The applicants have identified anthocyanin fractions that provide color characteristics closer to those provided by synthetic FD&C Blue No. 1 than any known natural blue colorant can provide, including Spirulina Blue.

An anthocyanin-containing vegetable or fruit juice may be obtained by pressing liquid out of the fruit or vegetable. An anthocyanin-containing vegetable or fruit extract may be obtained by washing a macerated fruit or vegetable with a solvent (e.g., water, alcohol). Juices and extracts contain anthocyanins as well as many other naturally occurring compounds, including, for example, carbohydrates, acids, flavonoids, metal ions, phenolic acids, phenolic acid esters, and vitamins. The term, "vegetable or fruit juice or extract," is equivalent to the list of terms, "vegetable juice, fruit juice, vegetable extract, or fruit extract," and includes processed juices and extracts, including, for example, reconstituted juices and extracts, deodorized juices and extracts, and juices and extracts subjected to other processes for removing specific or broad classes of compounds.

"Fractionation" is the process of selecting and separating a portion of anthocyanins from the complex mixture of anthocyanins in an anthocyanin-containing vegetable or fruit juice or extract. The blue anthocyanin-containing colorant of the invention may be sourced from an anthocyanin-containing vegetable or fruit juice or extract that provides blue hues at high pH values. In some embodiments, the blue anthocyanin-containing colorant may be sourced from red cabbage, purple sweet potato, blue potato, purple carrot or black carrot, or a combination thereof.

A "fraction" is the product of fractionation. An "anthocyanin fraction" contains a mixture of anthocyanins that is different from the mixture of anthocyanins in the anthocyanin-containing juice or extract from which the fraction was separated. Anthocyanin fractions are separated from the juice or extract at a select pH based on differences in charge and polarity of the different anthocyanin molecules present.

A "select pH" is a pH of 2 or higher, e.g. a pH in a range of about 2 to about 9, both in the context of separating and performing color characterization of anthocyanins. In other embodiments the pH may be at a pH of 3 or higher, 4 or higher, 5 or higher, 6 or higher,or 7 or higher, e.g., a pH in one of the following respective ranges, i.e., about 3 to about 9, about 4 to about 9, about 5 to about 9, about 6 to about 9 or about 7 to about 9.

One embodiment of the invention is directed to a natural blue anthocyanin-containing colorant sourced from vegetable, fruit or combinations thereof, comprising a selectively separated mixture of anthocyanins, wherein the colorant when in an aqueous solution at pH 8.0 has a maximum absorbance of 615 nm to 635 nm. The natural blue anthocyanin-containing colorant may comprise anthocyanins that have been selectively separated on an ion exchange column based on differences in charge and polarity of the anthocyanin molecules.

"Maximum absorbance," "lambda max," or "$\lambda_{max}$," is the wavelength in nanometers at which the maximum fraction of light is absorbed by a substance. In general, the maximum absorbance can be used as a characteristic value to compare substances when measured with a UV/Visible spectrophotometer or colorimeter.

References to "FD&C Blue No. 1" include the different names given to the identical synthetic blue colorant, Brilliant Blue FCF and European Commission E133. The lambda max of FD&C Blue No. 1 is 630 nm.

A "colorant" is any substance that imparts color by absorbing or scattering light at different wavelengths. A

"natural colorant" is a colorant that exists in or is produced by nature or is sourced therefrom. A "blue colorant" is a colorant that reflects light at wavelengths in the region of 450-495 nanometers and has a maximum absorbance ranging from 615 to 635 nanometers. A "natural anthocyanin-containing colorant" is a natural colorant comprising anthocyanins sourced from plants.

The natural anthocyanin-containing colorant is a composition that may comprise only anthocyanins that have been selectively separated (e.g., an isolated mixture) or may also include other plant components, carriers, e.g., water and/or excipients known for use with colorants. The composition may be in the form of a solid, e.g., a powder, or a liquid solution, e.g., an aqueous liquid.

In yet another embodiment, the invention is directed to the natural blue anthocyanin-containing colorant sourced from vegetable, fruit or a combination thereof, comprising a selectively separated mixture of anthocyanins, wherein the colorant when in an aqueous solution at pH 8.0 at a concentration in a range of 1 ppm to 1000 ppm has an h° value of 209 to 290. In another embodiment, the h° value may be about 225 to 250.

"Hue" refers to the color property that gives a color its name, for example, red, orange-red, blue, violet, etc.

"Chroma" is a color property indicating the purity of a color, where higher chroma is associated with greater purity of hue and less dilution by white, gray, or black.

"Value" is a color property indicating the lightness or darkness of a color, where higher value is associated with greater lightness.

The terms "color" and "color characteristics" are used interchangeably, and encompass color properties such as hue, chroma, and value and color model system parameters used to describe these properties, such as Commission Internationale de l'Eclairage CIE 1976 CIELAB color space L*a*b* values and CIELCH color space L*C*h° values. The CIELAB and CIELCH color models provide more perceptually uniform color spaces than earlier color models. Colorants are analyzed with a spectrophotometer, and CIELAB L*a*b* and CIELCH L*C*h° values are calculated from the spectral data. The L*a*b* and L*C*h° values provide a means of representing color characteristics and assessing the magnitude of difference between two colors. The CIELAB L*a*b* and CIELCH L*C*h° values presented herein, in all instances unless stated otherwise, were calculated from spectral data obtained from a Konica Minolta Spectrophotometer CM-3500d operated in transmittance mode, with CIE Standard Illuminant D65 and 10 degree observer angle.

L*a*b* values consist of a set of coordinate values defined in a three-dimensional Cartesian coordinate system. L* is the value, or lightness, coordinate. L* provides a scale of lightness from black (0 L* units) to white (100 L* units) on a vertical axis. a* and b* are coordinates related to both hue and chroma. a* provides a scale for greenness (- a* units) to redness (+ a* units), with neutral at the center point (0 a* units), on a horizontal axis. b* provides a scale for blueness (- b* units) to yellowness (+ b* units), with neutral at the center point (0 b* units), on a second horizontal axis perpendicular to the first horizontal axis. The three axes cross where L* has a value of 50 and a* and b* are both zero.

L*C*h° values consist of a set of coordinate values defined in a three-dimensional cylindrical coordinate system. L* is the value, or lightness, coordinate. L* provides a scale of lightness from black (0 L* units) to white (100 L* units) on a longitudinal axis. h° is the hue coordinate. h° is specified as an angle from 0° to 360° moving counterclockwise around the L* axis. Pure red has a hue angle of 0°, pure yellow has a hue angle of 90°, pure green has a hue angle of 180°, and pure blue has a hue angle of 270°. The C* coordinate represents chroma and is specified as a radial distance from the L* axis. C* provides a scale from achromatic, i.e., neutral white, gray, or black, at the L* axis (0 C* units) to greater purity of hue as the coordinate moves away from the L* axis (up to 100 or more C* units). C* and h° can be calculated from a* and b* using Equations 1 and 2:

$$C^* = (a^{*2} + b^{*2})^{0.5} \quad (1)$$

$$h^\circ = \arctan\left(\frac{b^*}{a^*}\right) \quad (2)$$

"Delta E," "$\Delta E_{ab}^*$," or "$\Delta E$" is a measure of the magnitude of total color difference between two colors represented in CIELAB L*a*b* color space. It has been reported that an experienced color observer cannot distinguish any difference between two colors when the $\Delta E$ is about 2.3 or less. The $\Delta E$ of two different colors with L*a*b* values, $L^*_1a^*_1b^*_1$ and $L^*_2a^*_2b^*_2$, is calculated using Equation 3:

$$\Delta E_{ab}^* = \sqrt{(L^*_1 - L^*_2)^2 + (a^*_1 - a^*_2)^2 + (b^*_1 - b^*_2)^2} \quad (3)$$

The CIELAB L*a*b* and CIELCH L*C*h° values of FD&C Blue No. 1 at seven different concentrations in aqueous

solution are presented in Table 1.

Table 1

| Concentration | L* | a* | b* | C* | h° |
|---|---|---|---|---|---|
| 1000 ppm | 10.49 | 15.82 | -44.99 | 47.69 | 289.37 |
| 500 ppm | 24.07 | 9.80 | -58.18 | 59.00 | 279.56 |
| 100 ppm | 52.43 | -29.57 | -57.38 | 64.55 | 242.74 |
| 50 ppm | 63.64 | -43.71 | -48.31 | 65.14 | 227.86 |
| 10 ppm | 84.25 | -37.23 | -23.42 | 43.99 | 212.17 |
| 5 ppm | 90.65 | -24.40 | -14.28 | 28.27 | 210.33 |
| 1 ppm | 97.69 | -6.43 | -3.57 | 7.36 | 209.02 |

These L*a*b* and L*C*h° values for FD&C Blue No. 1 represent the ideal target values for a natural blue colorant alternative to FD&C Blue No. 1. Natural blue colorants having L*a*b* values that fall within a ΔE of 2.3 or less from these target values would be expected to provide color characteristics sufficiently similar to those provided by FD&C Blue No. 1 that a human eye could not distinguish the difference in color provided by the natural colorant versus the synthetic. Clearly, the closer the L*a*b* values for a natural blue colorant come to the synthetic target values (i.e., yielding smaller values of ΔE), the better replacement the natural blue colorant will be for FD&C Blue No. 1 in an edible application.

Figure 1 shows two perspectives of a three dimensional representation of the L*a*b* values for aqueous solutions of FD&C Blue No. 1 at the seven concentrations reported in Table 1, connected by line segments. Figure 2 shows two perspectives of a three dimensional representation of the L*C*h° values for aqueous solutions of FD&C Blue No. 1 at the seven concentrations reported in Table 1, connected by line segments.

Mathematical models can be generated to represent the color characteristics provided by FD&C Blue No. 1 at any concentration in the L*a*b* and L*C*h° color spaces. For example, the color characteristics may be represented by a segmented line model connecting the L*a*b* or L*C*h° data points of Table 1. A line (L) connecting two points ($P^1$ and $P^2$) representing two different concentrations of FD&C Blue No. 1 in L*a*b* space can be calculated with the following Equation 4:

$$L = \{P_1 + t * (P_2 - P_1)\} \quad (4)$$

wherein $P_1$ is ($L^*_1$, $a^*_1$, $b^*_1$); $P_2$ is ($L^*_2$, $a^*_2$, $b^*_2$); and t is any real number.

Consequently, a segmented line model for FD&C Blue No. 1 in L*a*b* color space can be interpolated based on the L*a*b* values for the seven different concentration points using Equation 4 as follows:

For concentrations between 500 and 1000 ppm, 0 < t < 1:

$$L^* = 10.49 + 13.58 * t$$

$$a^* = 15.82 + -6.02 * t$$

$$b^* = -44.99 + -13.19 * t$$

For concentrations between 100 and 500 ppm, 0 < t < 1:

$$L^* = 24.07 + 28.36 * t$$

$$a^* = 9.80 + -39.37 * t$$

$$b^* = -58.18 + 0.80 * t$$

For concentrations between 50 and 100 ppm, 0 < t < 1:

$$L^* = 52.43 + 11.21 * t$$

$$a^* = -29.57 + -14.14 * t$$

$$b^* = -57.38 + 9.07 * t$$

For concentrations between 10 and 50 ppm, 0 < t < 1:

$$L^* = 63.64 + 20.61 * t$$

$$a^* = -43.71 + 6.48 * t$$

$$b^* = -48.31 + 24.89 * t$$

For concentrations between 5 and 10 ppm, 0 < t <1:

$$L^* = 84.25 + 6.40 * t$$

$$a^* = -37.23 + 12.83 * t$$

$$b^* = -23.42 + 9.14 * t$$

For concentrations between 1 and 5 ppm, 0 < t < 1:

$$L^* = 90.65 + 7.04 * t$$

$$a^* = -24.40 + 17.97 * t$$

$$b^* = -14.28 + 10.71 * t$$

The segmented line model for FD&C Blue No. 1 in L*a*b* space is drawn in Figure 1.

In addition, colors having L*a*b* values falling within a specific ΔE range of the FD&C Blue No. 1 model can be mathematically modeled in L*a*b* color space. Selecting a specific ΔE value, e.g., 3, with respect to FD&C Blue No. 1 and plotting that ΔE in L*a*b* color space results in a tube-like structure around the FD&C Blue No. 1 model, as shown in Figure 3. It is noted that any color with a ΔE value of about 2.3 or less from any point on the model will not be distinguishable from the color provided by FD&C Blue No. 1.

To determine whether a point ($X_0$) in L*a*b* color space falls within a specific ΔE value from the FD&C Blue

No. 1 model, the minimum distance, $d_{min}$, between the point and the model (represented by line segment $X_1$ to $X_2$) must be calculated.

Equation 5 can be used to calculate $d_{min}$:

$$d_{min} = \frac{\left|(x_0 - x_1) \times (x_0 - x_2)\right|}{\left|x_2 - x_1\right|} \qquad (5)$$

wherein x denotes the cross product of two vectors and vertical bars denote the magnitude of a vector expression. If the value of $d_{min}$ is less than or equal to the chosen ΔE value, then the point in L*a*b* color space falls within that specific ΔE value from the FD&C Blue No. 1 model.

For example, it may be determined whether Spirulina Blue provides a color having a ΔE of 12 or less compared to the color provided by FD&C Blue No. 1. Table 2 shows the color characteristics provided by Spirulina Blue, a known natural blue colorant, at two different concentrations in aqueous solution:

Table 2

| Concentration | L* | a* | b* | C* | h° |
|---|---|---|---|---|---|
| (404.8 mg/L) | 69.97 | -29.69 | -43.56 | 52.72 | 253.72 |
| (206 mg/L) | 80.3 | -23.97 | -29.39 | 37.92 | 230.8 |

The $X_0$ for the 404.8 mg/L Spirulina Blue solution in L*a*b* color space is:

$$X_0 = (69.97, -29.69, -43.56)$$

The $X_0$ for the 206 mg/L Spirulina Blue solution in L*a*b* color space is:

$$X_0 = (80.3, -23.97, -29.39)$$

$X_1$ and $X_2$ are two points from the FD&C Blue No. 1 model at 10 ppm and 50 ppm concentration in an aqueous solution, respectively:

$$X_1 = (63.64, -43.71, -48.31)$$

$$X_2 = (84.25, -37.23, -23.24)$$

The $d_{min}$, calculated using Equation 5, is 12.4 for the 404.8 mg/L Spirulina Blue solution and 14.4 for the 206 mg/L Spirulina Blue solution. Therefore, the Spirulina Blue solutions do not provide a color having a ΔE of 12 or less compared to the color provided by FD&C Blue No. 1 in aqueous solution when measured against the segmented line defined by the L*a*b* values for 10 ppm and 50 ppm FD&C Blue No. 1 in aqueous solution.

Spectral characteristics of a number of different solutions of Spirulina Blue were determined as shown in Table 3.

Table 3

| **Spirulina Solutions Data** | | | | | | |
|---|---|---|---|---|---|---|
| **Data Name** | **ppm** | **L*(D65)** | **a*(D65)** | **b*(D65)** | **C*(D65)** | **h°(D65)** |
| 0.04% Spirulina | 400 | 67.69 | -30.25 | -45.87 | 54.94 | 236.6 |
| 0.03% Spirulina | 300 | 72.77 | -29.43 | -39.52 | 49.27 | 233.32 |
| 0.02% Spirulina | 200 | 78.87 | -25.56 | -30.99 | 40.17 | 230.49 |

(continued)

| Spirulina Solutions Data | | | | | | |
|---|---|---|---|---|---|---|
| Data Name | ppm | L*(D65) | a*(D65) | b*(D65) | C*(D65) | h°(D65) |
| 0.015% Spirulina | 150 | 82.98 | -21.82 | -25.29 | 33.4 | 229.22 |
| 0.01% Spirulina | 100 | 87.77 | -16.29 | -18.32 | 24.52 | 228.35 |
| 0.0075% Spirulina | 75 | 90.46 | -12.94 | -14.27 | 19.27 | 227.79 |
| 0.005% Spirulina | 50 | 93.23 | -9.26 | -10.13 | 13.72 | 227.59 |

The data for Blue Spirulina has been plotted in the color graphs shown in Figure 5 versus the FD&C Blue No.1 data.

Differences between the color characteristics provided by Spirulina Blue and FD&C Blue No. 1 are represented in Figure 5. Figure 5 shows the segmented line model of the color characteristics provided by FD&C Blue No. 1 in L*a*b* color space at concentrations from 1 ppm to 1000 ppm in aqueous solution, with the model surrounded by a tube representing the area of colors that differ from the colors provided by Blue No. 1 by a ΔE of 3 or less. For comparison, Figure 5 also shows a segmented line model of the color characteristics provided by Spirulina Blue in L*a*b* color space at concentrations from 50 ppm to 400 ppm in aqueous solution. The Spirulina Blue model does not intersect the Blue No. 1 model or associated tube at any point in L*a*b* color space.

The invention includes natural blue anthocyanin-containing colorants sourced from vegetable, fruit or combinations thereof, comprising a selectively separated mixture of anthocyanins, wherein at least one concentration of the colorant when in an aqueous solution at pH 8.0 provides color characteristics having a ΔE value of 12 or less compared to the color characteristics defined by the segmented line defined by the L*a*b* values of 5 ppm and 10 ppm FD&C Blue No. 1 in aqueous solution. In other embodiments the ΔE value may be less than 11, 10, 9, 8, 7, 6, 5, 4 or 3. The at least one concentration of colorant may also if desired be measured against a plurality of segmented lines defined by different concentrations of FD&C Blue No. 1in aqueous solution, e.g., 1 and 5 ppm, 10 ppm and 50 ppm, 50 ppm and 100 ppm, 100 ppm and 500 ppm, 500 ppm and 1000 ppm, or any combination selected therefrom. For example, while not required, the at least one concentration of a colorant may be defined as having a ΔE value of 12 or less for a first segmented line at 5 ppm to 10 ppm, a ΔE value of 8 or less for a segmented line at 1 to 5 ppm and ΔE value of 12 or less for a segmented line at 10 ppm to 50 ppm. However, if ΔE value is used to describe the colorant of the invention, only one segmented line is required to define the colorant.

While Spirulina Blue is the natural colorant considered to provide the closest color match to FD&C Blue No. 1, the natural blue anthocyanin-containing colorant sourced from vegetable, fruit or combinations thereof, comprising a selectively separated mixture of anthocyanins, is a better color match. In particular, when at least one concentration of the colorant is in an aqueous solution at pH 8.0, that colorant aqueous solution provides color characteristics matching a FD&C Blue No. 1 segmented line based on a series of aqueous solutions having differing concentrations of FD&C Blue No.1 defined in an L*a*b* color space, wherein matching means the at least one concentration of the colorant in an aqueous solution at pH of 8.0 has a ΔE value measured against the FD&C Blue No. 1 segmented line that is at least one unit less than a ΔE value for a Spirulina Blue segmented line defined in the same L*a*b* color space based on a series of aqueous solutions having differing concentrations of Spirulina Blue measured against FD&C Blue No. 1 segmented line. In other embodiments the ΔE value of the at least one concentration of the colorant in an aqueous solution at pH of 8.0 measured against the FD&C Blue No. 1 segmented line is at least 2, 3, 4, 5 or 6 units less than a ΔE value for a Spirulina Blue segmented line measured against FD&C Blue No. 1 segmented line. In still other embodiments the ΔE value of the at least one concentration of the colorant in an aqueous solution at pH of 8.0 measured against the FD&C Blue No. 1 segmented line is at least 7, 8, 9, 10 or 11 units less than a ΔE value for a Spirulina Blue segmented line measured against FD&C Blue No. 1 segmented line.

Various fruit and vegetable extracts containing anthocyanins were analyzed to identify a source of anthocyanins that would provide color characteristics closest to those provided by the synthetic blue colorant, FD&C Blue No. 1. Figure 4 shows a comparison of six different commercially available extracts of red cabbage, purple sweet potato, black carrot, red radish, purple corn, and grape in aqueous solution at five different pH values. Visually, it can be seen that anthocyanins from red radish, purple corn, and grape did not provide blue hues in aqueous solution at any pH in the range from pH 6 to pH 8. Anthocyanins from red cabbage, purple sweet potato and black carrot provided blue hues in aqueous solution at the higher end of the pH range.

Any anthocyanin-containing fruit or vegetable juice or extract that provides blue hues at pH values of 4 or higher may be used as a source of anthocyanins. In some embodiments, the anthocyanin fraction is isolated from an extract of red cabbage, purple sweet potato, blue potato, purple carrot or black carrot, or a combination thereof. Red cabbage works well and is readily available.

Selected fractions of anthocyanin-containing fruit and vegetable juices and extracts may be isolated using an ion exchange column or semi-preparative HPLC column. Suitable ion exchange media include cation and anion exchange media. Suitable semi-preparative HPLC columns include C-18 columns. In an embodiment, the ion exchange column is activated with a solvent appropriate to the ion exchange media prior to loading of the vegetable or fruit juice or extract.

The anthocyanin-containing fraction is separated from the anthocyanin-containing vegetable or fruit juice or extract with a solvent at a pH of at least about 2, preferably at least about 4. In some embodiments, the anthocyanin fraction is separated with a solvent at a pH from about 2 to about 9. In yet another embodiment, the anthocyanin fraction is separated with a solvent at a pH from about 3 to about 9. In yet another embodiment, the anthocyanin fraction is separated with a solvent at a pH from about 4 to about 9. In yet another embodiment, the anthocyanin fraction is separated with a solvent at a pH from about 5 to about 9. In other embodiments, the anthocyanin fraction is separated with a solvent at a pH from about 6 to about 9. In still other embodiments, the anthocyanin fraction is separated with a solvent at a pH from about 7 to about 9.

Suitable solvents for eluting the selected fractions include methanol, acetonitrile, water, and mixtures thereof, depending on the polarity of the column media and the solubility of the anthocyanin-containing juice or extract. In some embodiments, the solvent is an aqueous methanol solution.

Suitable agents that may be added to the solvent to adjust pH include potassium phosphate, sodium hydroxide, and the like.

Additional anthocyanin fractions may be isolated by further fractionating the anthocyanin fraction using an ion exchange column or semi-preparative HPLC column. Suitable semi-preparative HPLC columns include C-18 columns.

In an embodiment, an isolated fraction or combination thereof provides a natural blue anthocyanin-containing colorant comprising anthocyanins selectively separated on an ion exchange column based on differences in polarity of the anthocyanin molecules, sourced from anthocyanin containing vegetable, fruit or extracts thereof.

In yet another embodiment, the natural blue anthocyanin-containing colorant of the invention is isolated from a natural vegetable or fruit source of anthocyanins by selective separation on an ion exchange column, the natural blue anthocyanin-containing colorant made by the process comprising the steps of:

a) loading an activated ion exchange column with an anthocyanin-containing vegetable or fruit juice or extract, or a combination thereof;
b) washing the loaded column with an aqueous solution of pH 8;
c) eluting a first fraction with a 25% v/v methanol solution at pH 8; and
d) eluting the natural blue anthocyanin-containing colorant with a 70% v/v methanol solution at pH 8 and, optionally, purifying and concentrating the natural blue anthocyanin-containing colorant.

In yet another embodiment, the natural blue anthocyanin-containing colorant of the invention is isolated from a natural source of anthocyanins by selective separation on an ion exchange column, the natural blue anthocyanin-containing colorant made by the process comprising the steps of:

a) loading an activated ion exchange column with an anthocyanin-containing vegetable or fruit juice or extract, or a combination thereof;
b) washing the loaded column with an aqueous solution of pH 6;
c) eluting a first fraction with a 25% v/v methanol solution at pH 6;
d) washing the loaded column with an aqueous solution of pH 7;
e) eluting a second fraction with a 25% v/v methanol solution at pH 7;
f) washing the loaded column with an aqueous solution of pH 8;
g) eluting a third fraction with a 25% v/v methanol solution at pH 8; and
h) eluting the natural blue anthocyanin-containing colorant with a 70% v/v methanol solution at pH 8 and, optionally, purifying and concentrating the natural blue anthocyanin-containing colorant.

In yet another embodiment, the natural blue anthocyanin-containing colorant of the invention comprises red cabbage anthocyanins selectively separated with semi-preparative HPLC, wherein the colorant when in an aqueous solution at pH 8.0 has a maximum absorbance of 620 nm to 635 nm..

Isolated anthocyanin-containing fractions may be used as colorants, or may be further processed by, for example, purification, concentration, deodorization, or color stabilization.

The natural blue anthocyanin-containing colorant may be applied to or incorporated into all types of edible products, including foods for human and animal consumption, beverages, and pharmaceutical products. Examples of edible products include pet food and treats, dry goods (e.g., rice, grains, and cereals), soups and sauces, confectionery products (e.g., chocolates, sugar and sugarless candies of all types, candy bars, chewing gum, and dragees), dessert products (e.g., pudding, frosting, icing, and toppings), baked goods (e.g., cakes, cookies, wafers, and biscuits), dairy

products (e.g., yogurt, whipped cream, and cheese), beverages (e.g., dairy-based drinks, waters, juices, teas, and sodas), snack products (e.g., crackers, snack bars, pretzels, and chips), and pharmaceutical forms (e.g., tablets, suspensions, chewables, and syrups). The natural blue anthocyanin-containing colorant may also be incorporated into food grade colorant compositions, coatings, and inks. In an embodiment, the blue anthocyanin-containing colorant is included in a coating or ink applied to a surface of a confectionery product. In another embodiment, the blue anthocyanin-containing colorant is included in a coating or ink applied to a surface of a confectionery product, wherein the confectionery product is a confectionery center with a soft panned or hard panned sugar-based coating. In yet another embodiment, the blue anthocyanin-containing colorant is included in a coating or ink applied to a surface of a confectionery product, wherein the confectionery product is a confectionery center with a soft panned or hard panned sugarless coating.

In an embodiment, a natural blue anthocyanin-containing colorant is prepared by fractionating red cabbage extract using a strong cation exchange column. A first fraction is eluted with a first eluent solvent solution that is a mixture of 75% v/v aqueous 0.1 M potassium phosphate buffer at pH 8 and 25% v/v methanol. A second fraction is eluted with a second eluent solvent solution that is a mixture of 30% v/v 0.1 M potassium phosphate buffer at pH 8 and 70% v/v methanol.

In another embodiment, a natural blue anthocyanin-containing colorant is prepared by fractionating red cabbage extract using a strong cation exchange column. A first fraction is eluted with 75% v/v 0.1 M potassium phosphate buffer at pH 6 and 25% v/v methanol. A second fraction is eluted with 75% v/v 0.1 M potassium phosphate buffer at pH 7 and 25% v/v methanol. A third fraction is eluted with 75% v/v 0.1 M potassium phosphate buffer at pH 8 and 25% v/v methanol. A fourth fraction is eluted with 30% v/v 0.1 M potassium phosphate buffer at pH 8 and 70% v/v methanol.

In another embodiment, a natural blue anthocyanin-containing colorant is separated from red cabbage extract using a C-18 semi-preparative HPLC column.

The anthocyanin-containing colorants of the invention fulfill the long-felt need for natural blue colorants that provide color characteristics similar to those provided by the synthetic blue colorant, FD&C Blue No. 1.

Specific embodiments of the invention will now be demonstrated by reference to the following examples. It should be understood that these examples are disclosed solely by way of illustrating the invention and variations within the spirit of the invention are anticipated.

**EXAMPLE 1**

Fractionation of Red Cabbage Extract Using Strong Cation Exchange Cartridge

An SCX (Strong Cation Exchange) solid phase extraction cartridge from Phenomenex® (Torrance, CA) was activated using pure methanol. The cartridge was washed using 0.01% v/v acidified water. An aqueous solution of red cabbage extract was loaded into the cartridge and washed with 0.01% v/v acidified water. A potassium phosphate buffer (0.1 M) at pH 8 was passed through the cartridge. Fraction 1 was eluted and collected using a 25% v/v methanol buffered aqueous solution at pH 8. Fraction 2 was eluted and collected using a 70% v/v methanol buffered aqueous solution at pH 8.

Fractions 1 and 2 were acidified with 2-5 ml of 88% v/v formic acid. The methanol was removed using a rotary evaporator.

In order to remove any salts, Fraction 1 was loaded into a C-18 cartridge and eluted with 0.01% v/v acidified water. The eluent was collected in 0.01% v/v acidified water, and the residual methanol was evaporated. Fraction 2 was also passed through a C-18 cartridge using the same procedure outlined for Fraction 1.

The maximum UV/VIS wavelength absorbance and color characteristics provided by the red cabbage extract solution (RCE) and Fractions 1 and 2 were analyzed at different pH values as shown below in Table 3. The spectral data was obtained using a Konica Minolta Spectrophotometer CM-3500d using the transmittance mode, CIE Standard Illuminant D65 and a 10 degree observer angle.

Table 3

| pH | | $\lambda_{max}$ | L* | a* | b* | C* | h° |
|---|---|---|---|---|---|---|---|
| 6.0 | RCE | 552.80 | 93.86 | 2.48 | -2.73 | 3.69 | 312.31 |
| | Fraction 1 | 551.40 | 94.43 | 2.24 | -2.24 | 3.17 | 314.98 |
| | Fraction 2 | 553.60 | 93.63 | 2.64 | -3.29 | 4.22 | 308.79 |
| 6.6 | RCE | 560.80 | 92.86 | 1.74 | -3.89 | 4.27 | 249.07 |
| | Fraction 1 | 558.20 | 93.54 | 1.75 | -3.31 | 3.75 | 297.89 |
| | Fraction 2 | 565.60 | 92.62 | 1.59 | -4.46 | 4.73 | 289.62 |

(continued)

| pH | | $\lambda_{max}$ | L* | a* | b* | C* | h° |
|---|---|---|---|---|---|---|---|
| 7.0 | RCE | 596.80 | 92.65 | -0.49 | -4.10 | 4.13 | 263.14 |
| | Fraction 1 | 594.0 | 92.43 | -0.22 | -4.60 | 4.61 | 267.28 |
| | Fraction 2 | 599.80 | 92.07 | -1.17 | -5.11 | 5.24 | 257.10 |
| 7.6 | RCE | 612.0 | 92.10 | -3.23 | -4.62 | 5.64 | 235.00 |
| | Fraction 1 | 608.40 | 91.41 | -3.47 | -5.80 | 6.76 | 239.08 |
| | Fraction 2 | 616.40 | 91.62 | -4.17 | -5.68 | 7.05 | 233.67 |
| 8.0 | RCE | 612.40 | 91.17 | -5.05 | -5.77 | 7.67 | 228.82 |
| | Fraction 1 | 610.60 | 90.90 | -5.26 | -6.40 | 8.29 | 230.59 |
| | Fraction 2 | 619.40 | 91.56 | -5.80 | -5.81 | 8.21 | 225.04 |

Fraction 2 at pH 7.6 and pH 8.0 provided $\lambda_{max}$ values closest to that of synthetic FD&C Blue No. 1 ($\lambda_{max}$ = 630 nm), i.e., $\lambda_{max}$ values of 616.40 and 619.40, respectively.

$\Delta$E values may also be calculated to compare the color characteristics provided by Fraction 2 at pH 7.6 and pH 8.0 to those provided by synthetic FD&C Blue No. 1. The $\Delta$E values are equivalent to the minimum distances between the Fraction 2 color points in L*a*b* color space and the FD&C Blue No. 1 model. Therefore, Equation 5 is used to calculate the $d_{min}$, or $\Delta$E, values from the following data:

The $X_0$ for Fraction 2 at pH 7.6 in L*a*b* color space is:

$$X_0 = (91.62, -4.17, -5.68)$$

The $X_0$ for Fraction 2 at pH 8.0 in L*a*b* color space is:

$$X_0 = (91.56, -5.80, -5.81)$$

$X_1$ and $X_2$ are two points from the FD&C Blue No. 1 model:

$$X_1 = (90.65, -24.40, -14.28)$$

$$X_2 = (97.69, -6.43, -3.57)$$

The calculated $d_{min}$, or $\Delta$E, values are 6.7 for Fraction 2 at pH 7.6, and 6.0 for Fraction 2 at pH 8.0.

Figure 6 provides HPLC chromatograms at 520 nm detection of the red cabbage extract solution (RCE) and Fractions 1 and 2. Figure 6 shows that Fraction 2 has a higher concentration of the later-eluting peaks from the red cabbage extract solution.

**EXAMPLE 2**

Fractionation of Red Cabbage Extract Using Strong Cation Exchange Cartridge and Solvents of Different High pH Values

An SCX (Strong Cation Exchange) solid phase extraction cartridge from Phenomenex® (Torrance, CA) was used. A red cabbage extract diluted in 0.01% v/v acidified water (10-15 ml) was loaded into the cartridge and washed with 0.01% v/v acidified water. A potassium phosphate buffer (0.1 M) at pH 6 was passed through the cartridge. Fraction 1 was eluted and collected using a 25% v/v methanol solution at pH 6. A potassium phosphate buffer (0.1 M) at pH 7 was passed through the cartridge. Fraction 2 was eluted and collected using a 25% v/v methanol solution at pH 7. A

potassium phosphate buffer (0.1 M) at pH 8 was passed through the cartridge. Fraction 3 was eluted and collected using a 25% v/v methanol solution at pH 8. Fraction 4 was eluted and collected using a 70% v/v methanol solution at pH 8.

Fractions 1 to 4 were acidified with 20% v/v formic acid. The methanol was removed using a rotary evaporator.

In order to wash the salts, Fraction 1 was loaded into a C-18 cartridge and eluted with 0.01% v/v acidified water. The eluent was collected in 0.01% v/v acidified water, and the residual methanol was evaporated. Fractions 2 to 4 were also passed through a C-18 cartridge using the same procedure outlined for Fraction 1.

The maximum UV/VIS wavelength absorbance and color characteristics provided by the red cabbage extract solution (RCE) and Fractions 1 to 4 were analyzed at different pH values as shown below in Table 4.

Table 4

| pH | | $\lambda_{max}$ | L* | a* | b* | C* | h° |
|---|---|---|---|---|---|---|---|
| 6.0 | RCE | 553.0 | 93.08 | 3.10 | -3.52 | 4.69 | 311.40 |
| | Fraction 1 | 549.8 | 95.21 | 1.64 | -1.33 | 2.11 | 320.92 |
| | Fraction 2 | 552.4 | 94.75 | 1.99 | -1.96 | 2.79 | 315.53 |
| | Fraction 3 | 552.0 | 94.42 | 2.19 | -2.25 | 3.13 | 314.20 |
| | Fraction 4 | 554.2 | 92.41 | 3.49 | -4.46 | 5.66 | 307.99 |
| 7.0 | RCE | 596.0 | 91.07 | -0.77 | -5.72 | 5.77 | 262.31 |
| | Fraction 1 | 592.6 | 93.37 | -0.12 | -3.36 | 3.36 | 267.91 |
| | Fraction 2 | 591.6 | 92.59 | 0.19 | -4.35 | 4.36 | 272.54 |
| | Fraction 3 | 594.4 | 92.32 | -0.38 | -4.62 | 4.63 | 265.34 |
| | Fraction 4 | 601.8 | 90.65 | -1.96 | -6.52 | 6.81 | 253.30 |
| 8.0 | RCE | 612.6 | 90.00 | -6.20 | -6.84 | 9.23 | 227.77 |
| | Fraction 1 | 606.6 | 91.05 | -4.44 | -5.87 | 7.36 | 232.93 |
| | Fraction 2 | 608.8 | 90.28 | -5.40 | -7.14 | 8.95 | 232.86 |
| | Fraction 3 | 611.6 | 90.21 | -5.92 | -7.16 | 9.29 | 230.42 |
| | Fraction 4 | 622.2 | 90.08 | -7.87 | -7.20 | 10.67 | 222.43 |

Fraction 4 at pH 8.0 provided a $\lambda_{max}$ value closest to that of synthetic FD&C Blue No. 1 ($\lambda_{max}$ = 630 nm), i.e., a $\lambda_{max}$ value of 622.2.

A $\Delta E$ value may also be calculated to compare the color characteristics provided by Fraction 4 at pH 8.0 to those provided by synthetic FD&C Blue No. 1. The $\Delta E$ value is equivalent to the minimum distance between the Fraction 4 color point in L*a*b* color space and the FD&C Blue No. 1 model. Therefore, Equation 5 is used to calculate the $d_{min}$, or $\Delta E$, value from the following data:

The $X_0$ for Fraction 4 at pH 8.0 in L*a*b* color space is:

$$X_0 = (90.08, -7.87, -7.20)$$

$X_1$ and $X_2$ are two points from the FD&C Blue No. 1 model:

$$X_1 = (90.65, -24.40, -14.28)$$

$$X_2 = (97.69, -6.43, -3.57)$$

The calculated $d_{min}$, or $\Delta E$, value is 6.7 for Fraction 4 at pH 8.0. Figure 7 provides the HPLC chromatograms at 520 nm detection of the red cabbage extract solution (RCE) and Fractions 1 to 4. Figure 7 shows that Fraction 4 has a higher concentration of the later-eluting peaks from the red cabbage extract solution.

**EXAMPLE 3**

Separation of Red Cabbage Extract Peak Groups Using Semi-Preparative HPLC

Fractions associated with two specific groups of peaks, as shown in the chromatogram of Figure 8, may be separated and collected from red cabbage extract solution using semi-preparative HPLC. The red cabbage extract solution was loaded onto a C-18 semi-preparative HPLC cartridge and two fractions, the 520-nm Fraction ($\lambda_{max}$ = 524 nm) and the 530-nm Fraction ($\lambda_{max}$ = 532 nm), were eluted using an acidic acetonitrile and water gradient. The residual acetonitrile was evaporated from each fraction with a rotary evaporator.

Color characterization was performed after adjusting the concentrations of the fractions and mixing separate fraction aliquots with buffer to produce five aliquots at pH 6, 6.6, 7, 7.6, and 8. The maximum UV/VIS wavelength absorbance and color characteristics of the 520-nm and 530-nm Fraction aliquots were analyzed, and the results are provided in Table 5.

Table 5

| Fraction | pH | $\lambda_{max}$ | Abs (Amu) | L* | a* | b* | C* | h° |
|---|---|---|---|---|---|---|---|---|
| 520-nm (107.71 mg/L) | 1-2 | 524 | 2.161 | 80.05 | 33.41 | -6.17 | 33.97 | 349.54 |
| | 6.0 | ND[1] | ND | 95.95 | 0.95 | -0.72 | 1.19 | 322.87 |
| | 6.6 | ND | ND | 95.64 | 0.80 | -1.08 | 1.34 | 306.49 |
| | 7.0 | 585.80 | 0.2 | 99.32 | 0.48 | -2.56 | 2.60 | 280.71 |
| | 7.6 | 602.00 | 0.389 | 92.56 | -1.72 | -4.70 | 5.01 | 249.91 |
| | 8.0 | 603.80 | 0.488 | 92.09 | -3.15 | -5.17 | 6.06 | 238.68 |
| 530-nm (55.60 mg/L) | 1-2 | 538 | 0.752 | 89.83 | 13.86 | -5.80 | 15.02 | 337.31 |
| | 6.0 | 554.40 | 0.610 | 89.27 | 6.07 | -8.06 | 10.10 | 306.98 |
| | 6.6 | 587.00 | 0.707 | 87.64 | 1.43 | -9.96 | 10.06 | 278.14 |
| | 7.0 | 599.60 | 0.848 | 86.88 | -2.85 | -10.96 | 11.33 | 255.45 |
| | 7.6 | 621.80 | 1.156 | 87.67 | -5.44 | -9.90 | 11.30 | 241.23 |
| | 8.0 | 621.00 | 1.294 | 86.39 | -11.79 | -11.98 | 16.81 | 225.45 |

[1] ND indicates that the absorbance spectra of the sample did not show a maximum peak in the visible range.
The 530-nm Fraction has a maximum absorbance of about 621 nm at pH 7.6 and pH 8.0 and provides a $\lambda_{max}$ closest to that of synthetic FD&C Blue No. 1 ( $\lambda_{max}$ = 630 nm).

$\Delta$E values may also be calculated to compare the color characteristics provided by the 530-nm Fraction at pH 7.6 and pH 8.0 to those provided by synthetic FD&C Blue No. 1. The $\Delta$E values are equivalent to the minimum distances between the 530-nm Fraction color points in L*a*b* color space and the FD&C Blue No. 1 model. Therefore, Equation 5 is used to calculate the $d_{min}$, or $\Delta$E, values from the following data:

The $X_0$ for the 530-nm Fraction at pH 7.6 in L*a*b* color space is:

$$X_0 = (87.67, -5.44, -9.90)$$

The $X_0$ for the 530-nm Fraction at pH 8.0 in L*a*b* color space is:

$$X_0 = (86.39, -11.79, -11.98)$$

$X_1$ and $X_2$ are two points from the FD&C Blue No. 1 model:

$$X_1 = (84.25, -37.23, -23.42)$$

$$X_2 = (90.65, -24.40, -14.28)$$

The calculated $d_{min}$, or ΔE, values are 12.1 for the 530-nm Fraction at pH 7.6, and 9.9 for the 530-nm Fraction at pH 8.0.

Figure 9 provides a visual comparison of the 520-nm and 530-nm Fractions at different pH values. The concentration of the 520-nm Fraction is 107.7 mg/L (Cyn-3-glu) and the concentration of the 530-nm Fraction is 55.6 mg/L (Cyn-3-glu). At neutral and higher pH, it can be seen that the 530-nm Fraction provides two to four times the chroma (as measured by C*) of the 520-nm Fraction at half the colorant concentration.

Figure 10 provides the HPLC chromatograms at 520 nm detection of the red cabbage extract solution and the 520-nm and 530-nm Fractions. Figure 10 indicates that each fraction contains three distinct anthocyanin compounds.

**COMPARATIVE EXAMPLE 1**

Several different concentrations of the red cabbage anthocyanin solution disclosed in the Examples of WO 2004/012526 were prepared at pH of 8.0. There was no fractionation conducted to separate and collect separated anthocyanin-containing colorants. The maximum absorbance of the resulting solutions was 610 nm. The color was not considered an acceptable match for the color of FD&C Blue No. 1.

**Claims**

1. A natural blue anthocyanin-containing colorant sourced from vegetable, fruit or combinations thereof, comprising a selectively separated mixture of anthocyanins, wherein the colorant when in an aqueous solution at pH 8.0 has a maximum absorbance of 615 nm to 635 nm.

2. The natural blue anthocyanin-containing colorant according to claim 1, wherein the colorant when in an aqueous solution at pH 8.0 at a concentration in a range of 1 ppm to 1000 ppm has an h° value of 209 to 290.

3. A natural blue anthocyanin-containing colorant according to claim 1 or claim 2, wherein at least one concentration of the colorant when in an aqueous solution at pH 8.0 provides color characteristics having a ΔE value of 12 or less compared to the color characteristics defined by the segmented line defined by the L*a*b* values of 5 ppm and 10 ppm FD&C Blue No. 1 in aqueous solution.

4. The natural blue anthocyanin-containing colorant according to claim 3, wherein the h° value of the at least one concentration of colorant in aqueous solution is in a range from 209 to 250.

5. The natural blue anthocyanin-containing colorant of any one of claims 1 to 4, wherein the source of the anthocyanins is selected from the group consisting of red cabbage, purple sweet potato, blue potato, black carrot, purple carrot and combinations thereof.

6. The natural blue anthocyanin-containing colorant of claim 5, wherein the source of the anthocyanins is red cabbage.

7. A natural blue anthocyanin-containing colorant according to any one of the preceding claims, wherein at least one concentration of the colorant when in an aqueous solution at pH 8.0 provides color characteristics matching a FD&C Blue No.1 segmented line based on a series of aqueous solutions having differing concentrations of FD&C Blue No.1 defined in an L*a*b* color space, wherein matching means the at least one concentration of the colorant in an aqueous solution at pH of 8.0 has a ΔE value measured against the FD&C Blue No. 1 segmented line that is at least one unit less than a ΔE value for a Spirulina Blue segmented line defined in the same L*a*b* color space based on a series of aqueous solutions having differing concentrations of Spirulina Blue measured against the FD&C Blue No.1 segmented line.

8. A natural green colorant consisting essentially of a natural yellow colorant and the natural blue anthocyanin-containing colorant of any one of the preceding claims.

9. An edible product comprising the natural blue anthocyanin-containing colorant of any one of claims 1 to 7.

**10.** The edible product of claim 9, further comprising a natural yellow colorant.

**11.** The edible product of claim 9 or claim 10, wherein the product is a confectionery product.

**12.** The confectionery product of claim 11, further comprising a natural yellow colorant.

**13.** The confectionery product of claim 11, wherein the colorant is included in a coating applied to a surface of the confectionery product.

**14.** The confectionery product of claim 13, wherein the confectionery product is a confectionery center with a soft panned or hard panned sugar-based coating.

**15.** The confectionery product of claim 13, wherein the confectionery product is a confectionery center with a soft panned or hard panned sugarless coating.

**Patentansprüche**

**1.** Natürlicher blauer Anthocyanin-haltiger Farbstoff, der von Gemüse, Obst oder Kombinationen davon gewonnen wurde, umfassend ein selektiv getrenntes Gemisch von Anthocyaninen, wobei der Farbstoff, wenn er in einer wässrigen Lösung bei pH 8,0 vorliegt, eine maximale Absorbanz von 615 nm bis 635 nm hat.

**2.** Natürlicher blauer Anthocyanin-haltiger Farbstoff nach Anspruch 1, wobei der Farbstoff, wenn er in einer wässrigen Lösung bei pH 8,0 in einer Konzentration in einem Bereich von 1 ppm bis 1000 ppm vorliegt, einen h° Wert von 209 bis 290 hat.

**3.** Natürlicher blauer Anthocyanin-haltiger Farbstoff nach Anspruch 1 oder Anspruch 2, wobei wenigstens eine Konzentration des Farbstoffs, wenn er in einer wässrigen Lösung bei pH 8,0 vorliegt, Farbcharakteristiken mit einem ΔE-Wert von 12 oder weniger im Vergleich zu den Farbcharakteristiken bietet, die durch die segmentierte Linie definiert sind, die durch die L*a*b* Werte von 5 ppm und 10 ppm FD&C Blau Nr. 1 in wässriger Lösung definiert wird.

**4.** Natürlicher blauer Anthocyanin-haltiger Farbstoff nach Anspruch 3, wobei der h°-Wert der wenigstens einen Konzentration von Farbstoff in wässriger Lösung im Bereich von 209 bis 250 vorliegt.

**5.** Natürlicher blauer Anthocyanin-haltiger Farbstoff nach einem der Ansprüche 1 bis 4, wobei die Quelle der Anthocyanine aus der Gruppe bestehend aus Rotkohl, violetter Süßkartoffel, blauer Kartoffel, schwarzer Karotte, violetter Karotte und Kombinationen davon ausgewählt ist.

**6.** Natürlicher blauer Anthocyanin-haltiger Farbstoff nach Anspruch 5, wobei die Quelle der Anthocyanine Rotkohl ist.

**7.** Natürlicher blauer Anthocyanin-haltiger Farbstoff nach einem der vorherigen Ansprüche, wobei wenigstens eine Konzentration des Farbstoffs, wenn er in einer wässrigen Lösung bei pH 8,0 vorliegt, Farbcharakteristiken bietet, die mit einer segmentierten Linie von FD&C Blau Nr. 1 übereinstimmen, auf der Basis einer Serie von wässrigen Lösungen mit unterschiedlichen Konzentrationen von FD&C Blau Nr. 1, definiert in einem L*a*b* Farbraum, wobei Übereinstimmung bedeutet, dass die wenigstens eine Konzentration des Farbstoffs in einer wässrigen Lösung bei pH 8,0 einen ΔE-Wert hat, gemessen anhand der segmentierten Linie von FD&C Blau Nr. 1, der wenigstens um eine Einheit kleiner ist als ein ΔE-Wert für eine segmentierte Spirulina-blaue Linie, definiert im selben L*a*b* Farbraum auf der Basis einer Serie von wässrigen Lösungen mit unterschiedlichen Konzentrationen von Spirulina-Blau, gemessen anhand der segmentierten Linie von FD&C Blau Nr. 1.

**8.** Natürlicher grüner Farbstoff, der im Wesentlichen aus einem natürlichen gelben Farbstoff und dem natürlichen blauen Anthocyanin-haltigen Farbstoff nach einem der vorherigen Ansprüche besteht.

**9.** Essbares Produkt, das den natürlichen blauen Anthocyanin-haltigen Farbstoff nach einem der Ansprüche 1 bis 7 enthält.

**10.** Essbares Produkt nach Anspruch 9, das ferner einen natürlichen gelben Farbstoff enthält.

11. Essbares Produkt nach Anspruch 9 oder Anspruch 10, wobei das Produkt ein Süßwarenprodukt ist.

12. Süßwarenprodukt nach Anspruch 11, das ferner einen natürlichen gelben Farbstoff enthält.

13. Süßwarenprodukt nach Anspruch 11, wobei der Farbstoff in einer Beschichtung enthalten ist, die auf eine Oberfläche des Süßwarenprodukts aufgebracht wird.

14. Süßwarenprodukt nach Anspruch 13, wobei das Süßwarenprodukt ein Süßwarenzentrum mit einer weich dragierten oder hart dragierten Beschichtung auf Zuckerbasis ist.

15. Süßwarenprodukt nach Anspruch 13, wobei das Süßwarenprodukt ein Süßwarenzentrum mit einer weich dragierten oder hart dragierten zuckerlosen Beschichtung ist.

**Revendications**

1. Colorant bleu naturel contenant des anthocyanines provenant d'un légume, d'un fruit ou leurs combinaisons, comprenant un mélange d'anthocyanines séparé sélectivement, dans lequel le colorant présente dans une solution aqueuse à pH 8,0 une absorbance maximale de 615 nm à 635 nm.

2. Colorant bleu naturel contenant des anthocyanines selon la revendication 1, dans lequel le colorant présente dans une solution aqueuse à pH 8,0 à une concentration dans une plage allant de 1 ppm à 1 000 ppm une valeur° h de 209 à 290.

3. Colorant bleu naturel contenant des anthocyanines selon la revendication 1 ou la revendication 2, dans lequel au moins une concentration du colorant dans une solution aqueuse à pH 8,0 permet d'obtenir des caractéristiques de couleur ayant une valeur ΔE de 12 ou moins en comparaison des caractéristiques de couleur définies par la ligne segmentée définie par les valeurs L*a*b* de 5 ppm et 10 ppm de FD&C Blue No. 1 dans une solution aqueuse.

4. Colorant bleu naturel contenant des anthocyanines selon la revendication 3, dans lequel la valeur° h de ladite au moins une concentration de colorant dans une solution aqueuse est dans une plage allant de 209 à 250.

5. Colorant bleu naturel contenant des anthocyanines selon l'une quelconque des revendications 1 à 4, dans lequel la source des anthocyanines est choisie dans le groupe constitué par le chou rouge, la patate douce violette, la pomme de terre bleue, la carotte noire, la carotte violette et leurs combinaisons.

6. Colorant bleu naturel contenant des anthocyanines selon la revendication 5, dans lequel la source des anthocyanines est le chou rouge.

7. Colorant bleu naturel contenant des anthocyanines selon l'une quelconque des revendications précédentes, dans lequel au moins une concentration du colorant dans une solution aqueuse à pH 8,0 permet d'obtenir des caractéristiques de couleur correspondant à une ligne segmentée FD&C Blue No.1 fondée sur une série de solutions aqueuses ayant différentes concentrations de FD&C Blue No.1 définies dans un espace de couleur L*a*b*, correspondant signifiant que ladite au moins une concentration du colorant dans une solution aqueuse à pH 8,0 a une valeur ΔE comparée à la ligne segmentée FD&C Blue No. 1 qui est au moins une unité de moins qu'une valeur ΔE pour une ligne segmentée Spirulina Blue définie dans le même espace de couleur L*a*b* fondée sur une série de solutions aqueuses ayant différentes concentrations de Spirulina Blue comparée à la ligne segmentée FD&C Blue No.1.

8. Colorant vert naturel essentiellement constitué par un colorant jaune naturel et le colorant bleu naturel contenant des anthocyanines selon l'une quelconque des revendications précédentes.

9. Produit comestible comprenant le colorant bleu naturel contenant des anthocyanines selon l'une quelconque des revendications 1 à 7.

10. Produit comestible selon la revendication 9, comprenant en outre un colorant jaune naturel.

11. Produit comestible selon la revendication 9 ou la revendication 10, dans lequel le produit est un produit de confiserie.

**12.** Produit de confiserie selon la revendication 11, comprenant en outre un colorant jaune naturel.

**13.** Produit de confiserie selon la revendication 11, dans lequel le colorant est compris dans un revêtement appliqué sur une surface du produit de confiserie.

**14.** Produit de confiserie selon la revendication 13, dans lequel le produit de confiserie est un centre de confiserie muni d’un revêtement à base de sucre mou ou dur.

**15.** Produit de confiserie selon la revendication 13, dans lequel le produit de confiserie est un centre de confiserie muni d’un revêtement sans sucre mou ou dur.

FIGURE 1

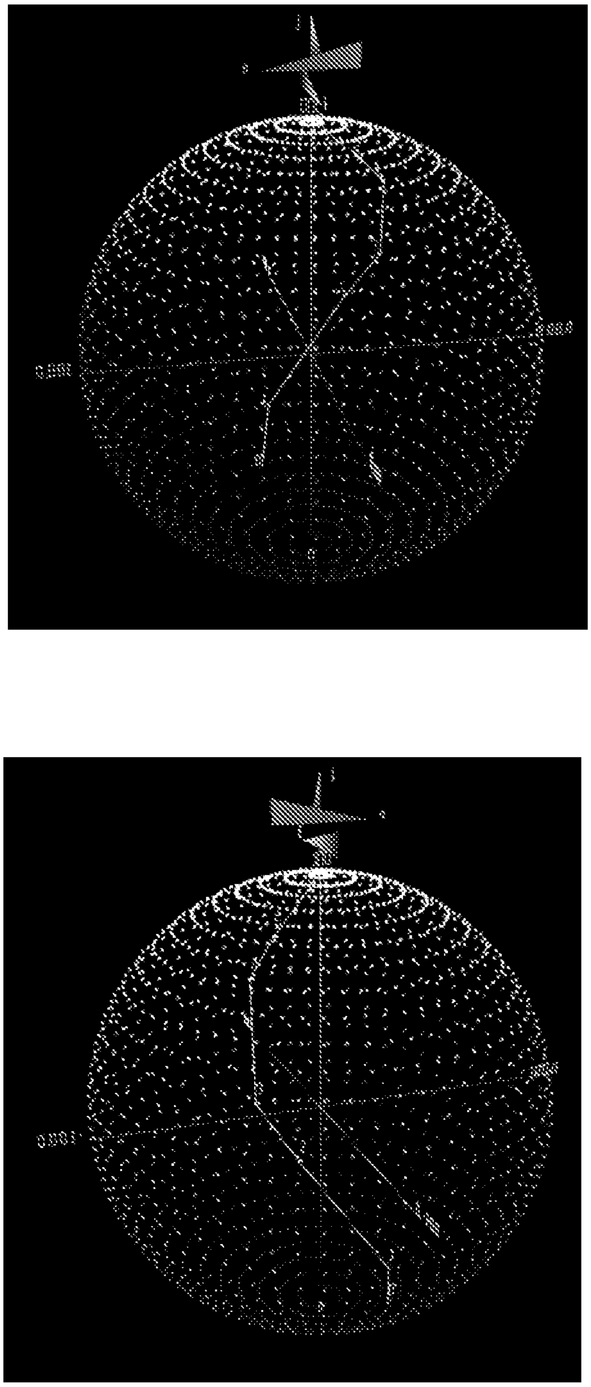

FIGURE 2

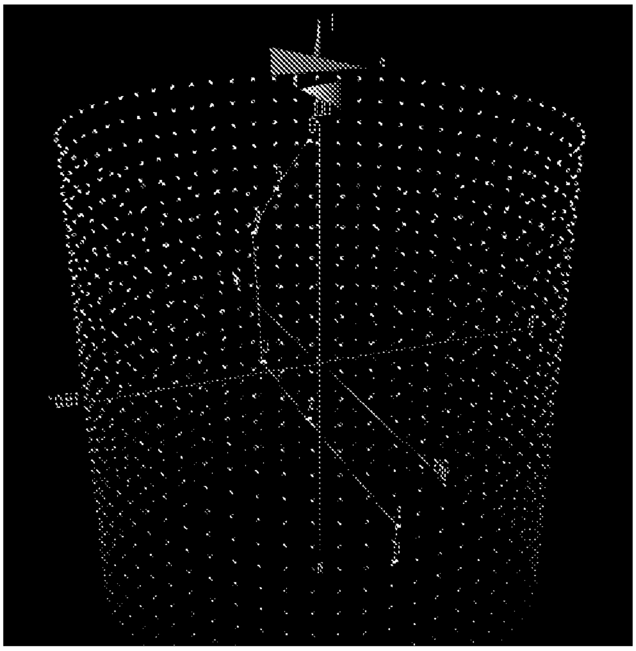

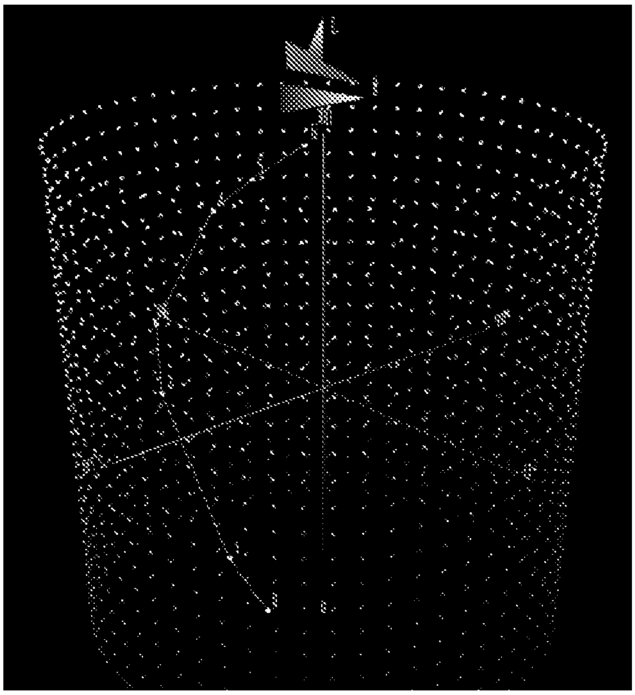

FIGURE 3

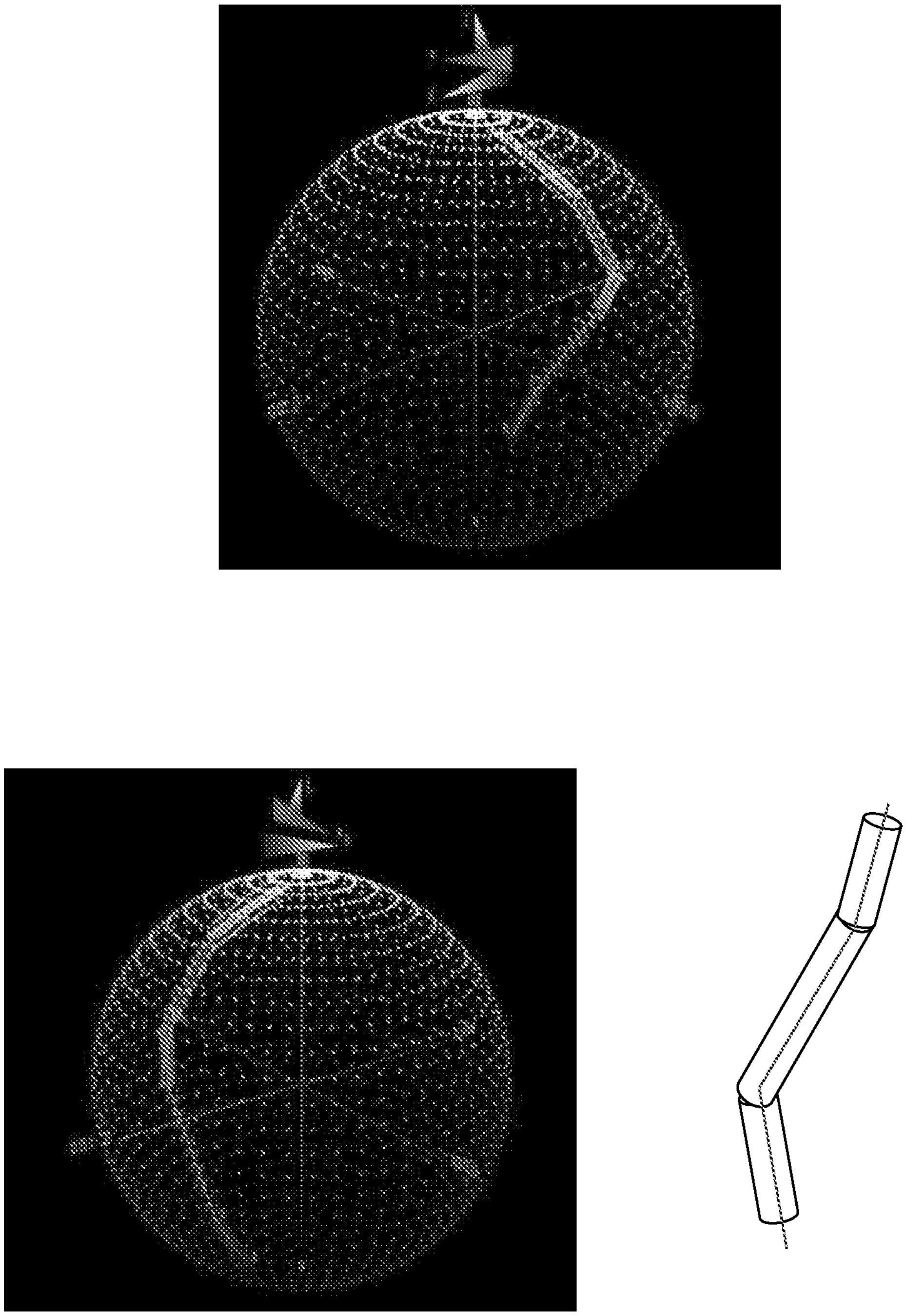

FIGURE 4

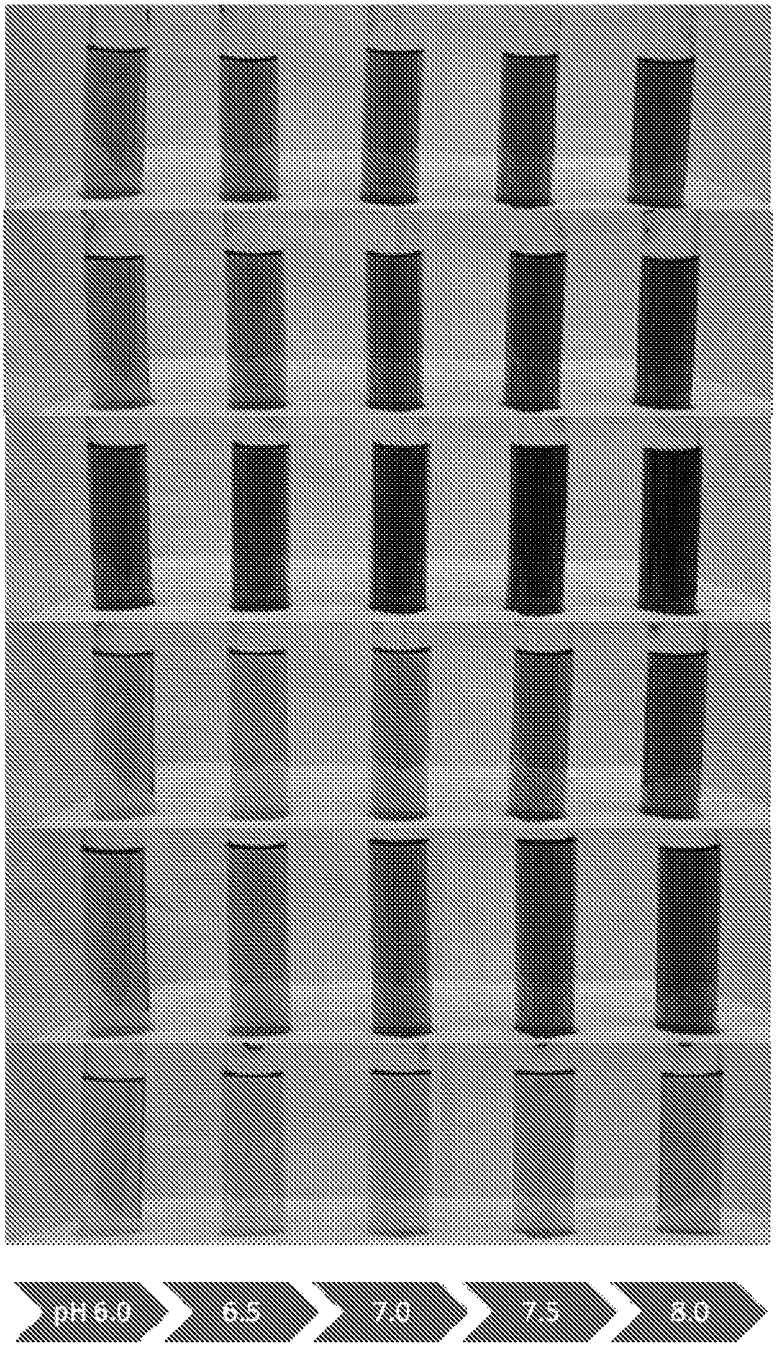


Red Cabbage

Purple Sweet Potato

Black Carrot

Red Radish

Purple Corn

Grape

FIGURE 5

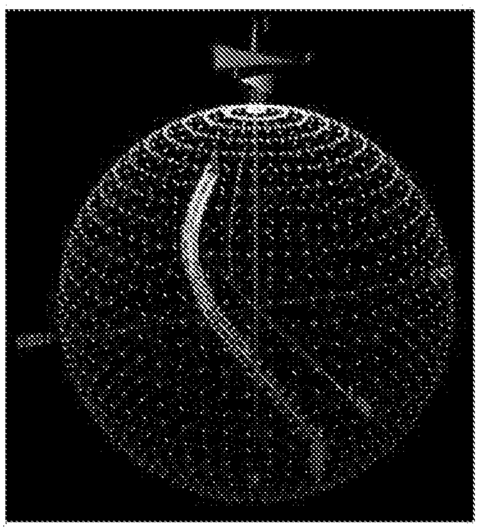

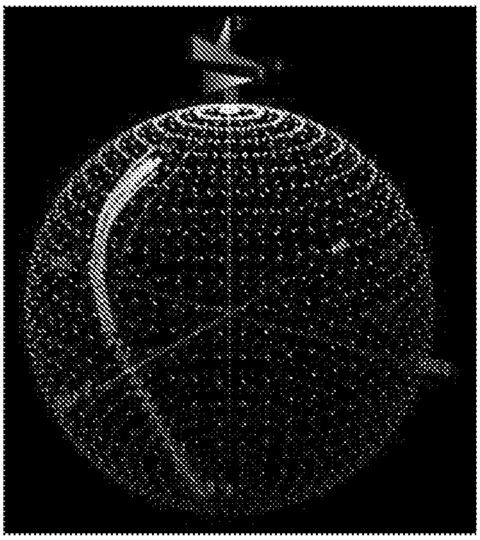

FIGURE 6

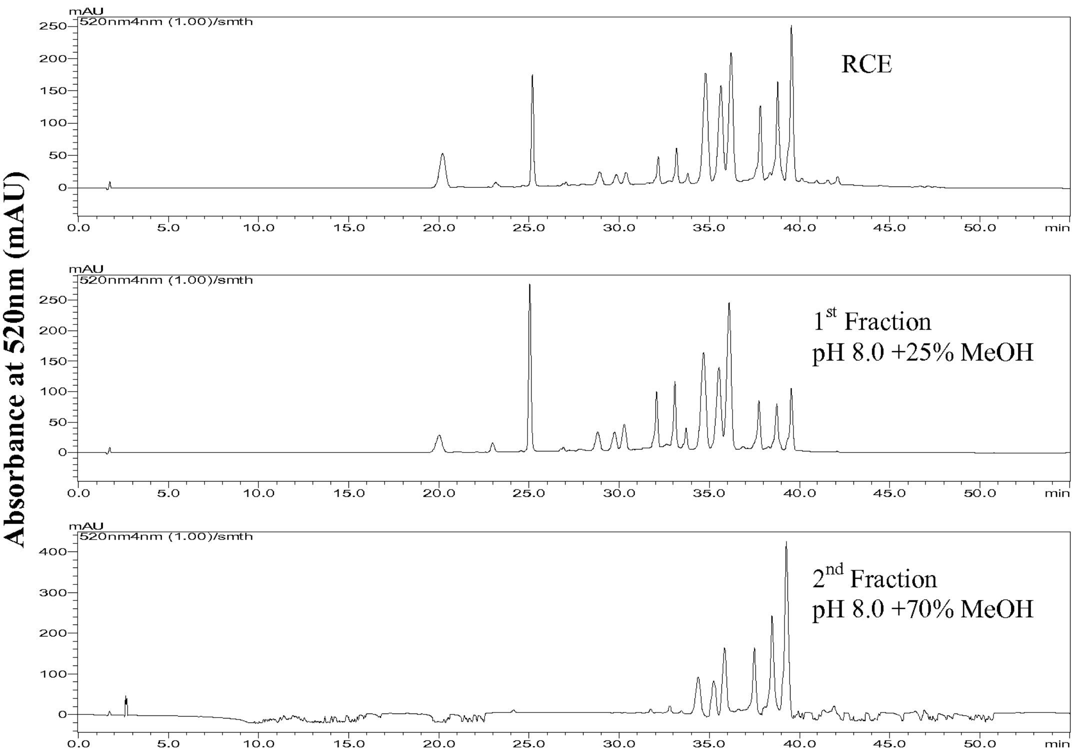
Absorbance at 520nm (mAU)
mAU
520nm4nm (1.00)/smth
RCE
1st Fraction
pH 8.0 +25% MeOH
2nd Fraction
pH 8.0 +70% MeOH
min

FIGURE 7

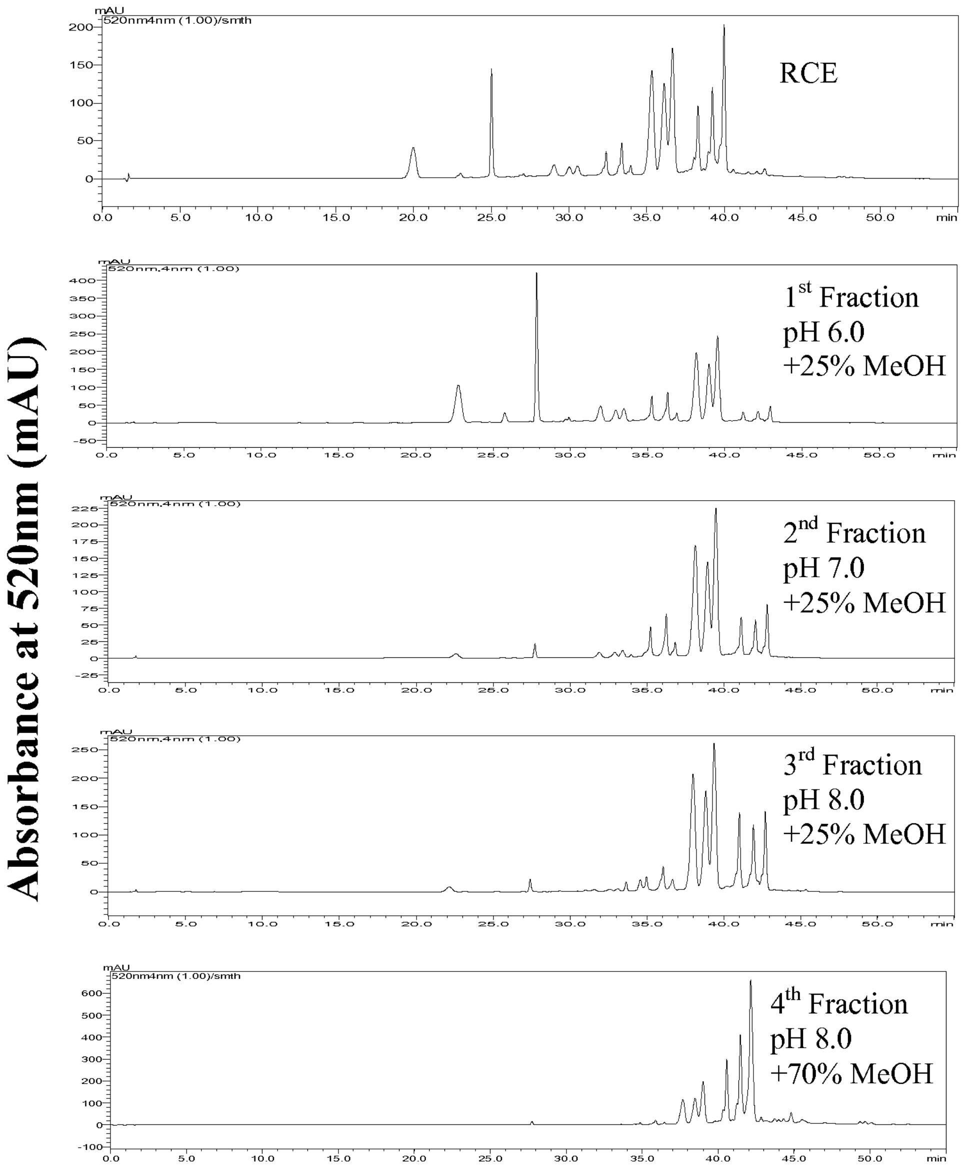
Absorbance at 520nm (mAU)
mAU
520nm4nm (1.00)/smth
RCE
520nm,4nm (1.00)
1st Fraction
pH 6.0
+25% MeOH
2nd Fraction
pH 7.0
+25% MeOH
3rd Fraction
pH 8.0
+25% MeOH
4th Fraction
pH 8.0
+70% MeOH
min

FIGURE 8

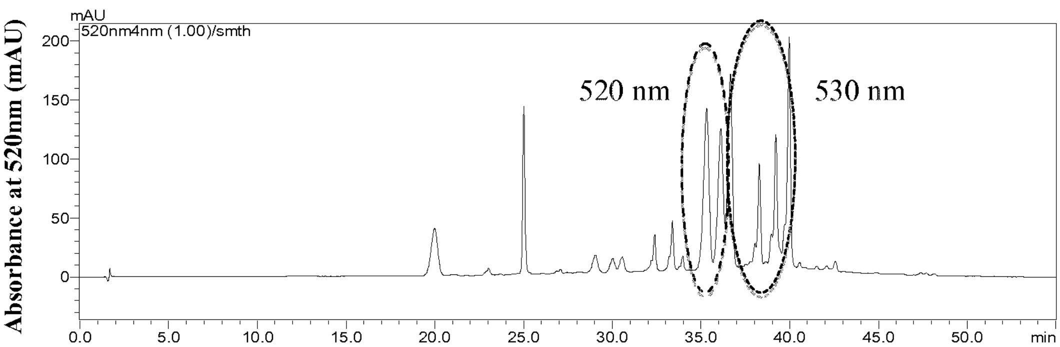
mAU
520nm4nm (1.00)/smth
Absorbance at 520nm (mAU)
200
150
100
50
0
520 nm
530 nm
0.0
5.0
10.0
15.0
20.0
25.0
30.0
35.0
40.0
45.0
50.0
min

FIGURE 9

| | | | | | | | | | |
|---|---|---|---|---|---|---|---|---|---|
| 520 pH 6.0 | 530 pH 6.0 | 520 pH 6.5 | 530 pH 6.5 | 520 pH 7.0 | 530 pH 7.0 | 520 pH 7.5 | 530 pH 7.5 | 520 pH 8.0 | 530 pH 8.0 |

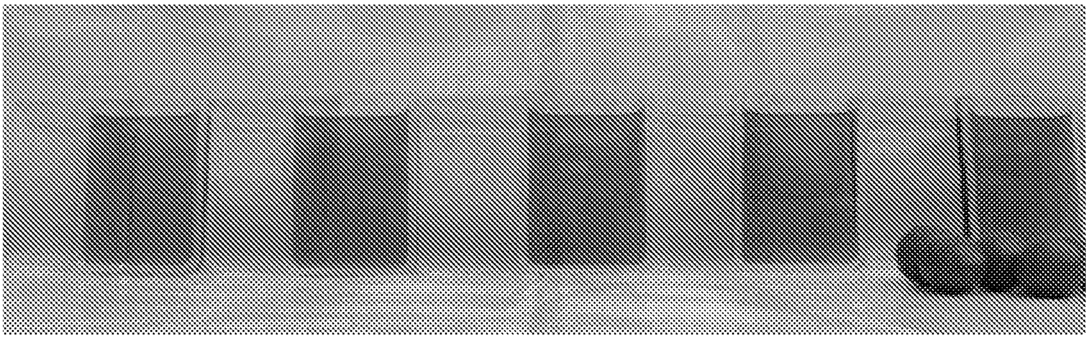

FIGURE 10

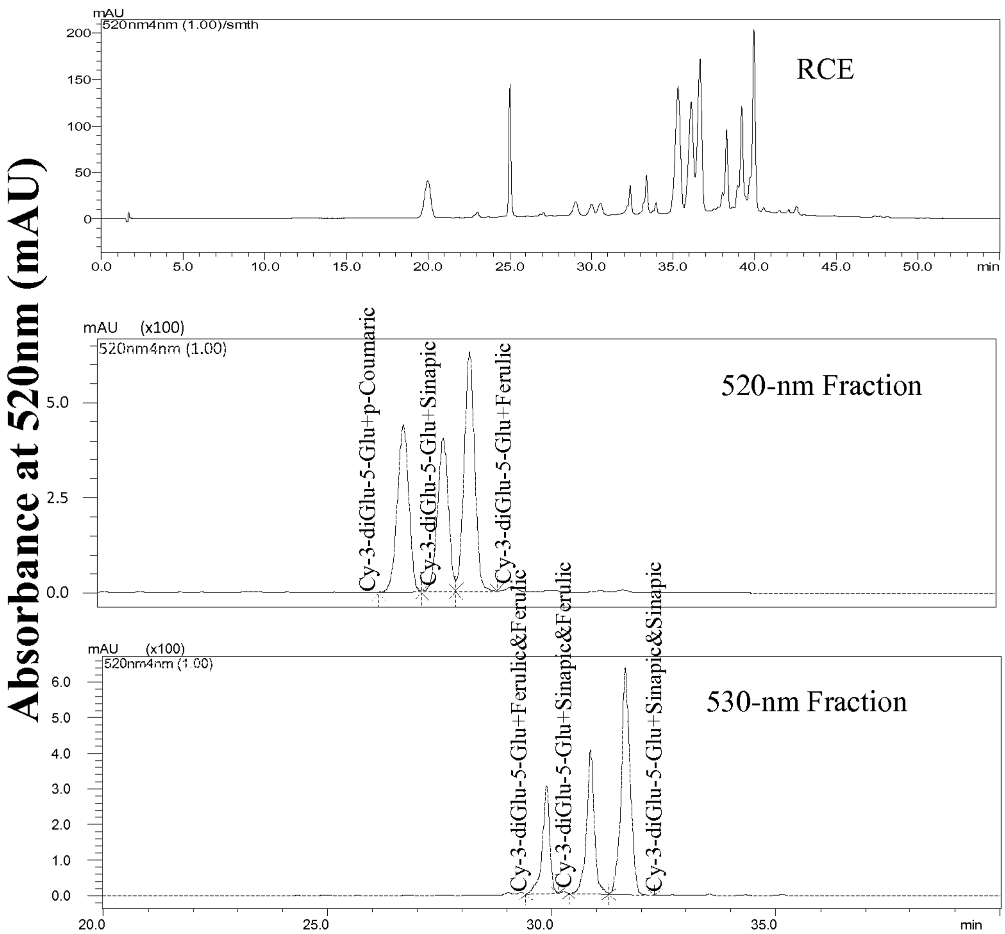
Absorbance at 520nm (mAU)
mAU
520nm4nm (1.00)/smth
RCE
min
mAU (x100)
520nm4nm (1.00)
520-nm Fraction
Cy-3-diGlu-5-Glu+p-Coumaric
Cy-3-diGlu-5-Glu+Sinapic
Cy-3-diGlu-5-Glu+Ferulic
mAU (x100)
520nm4nm (1.00)
530-nm Fraction
Cy-3-diGlu-5-Glu+Ferulic&Ferulic
Cy-3-diGlu-5-Glu+Sinapic&Ferulic
Cy-3-diGlu-5-Glu+Sinapic&Sinapic
min

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009100165 A2
- WO 2004012526 A
- WO 2010114568 A1


### Non-patent literature cited in the description


- *J. Chromatography A.,* 2007, vol. 1148, 38-45